# FASCICULE DE BREVET EUROPEEN

(11) **EP 1 097 699 B1**
(45) Date de publication et mention de la délivrance du brevet: **23.05.2007**
(21) Numéro de dépôt: 00402958.3
(22) Date de dépôt: 25.10.2000
(51) Int. Cl.: A61Q 1/06

(54) **Composition cosmétique contenant un ester d'acide gras hydroxyle**
Kosmetische Zubereitung Ester hydroxylierter Fettsäuren enthaltend
Cosmetic composition comprising hydroxylated fatty acid ester

(30) Priorité: 04.11.1999 FR 9913812
(43) Date de publication de la demande: 09.05.2001
(73) Titulaire: L'ORÉAL, 75008 Paris (FR)
(72) Inventeur: Arnaud, Pascal, 94240 - L'Hay les Roses (FR)
(74) Mandataire: Chantraine, Sylvie Hélène

(56) Documents cités:
- GB-A- 590 386
- US-A- 5 959 130
- FINETEX INC.: "Finsolv BCO-115 (Castor oil benzoate)" FINETEX HOMEPAGE: PRODUCT INDEX - TECHNICAL DATA SHEET, [en ligne] 1999, XP002144802 Extrait de l'Internet: <URL:http://www.finetexinc.com/FINSOLV%20B CO-115.html> [extrait le 2000-08-11]

## Description

La présente invention se rapporte à une composition de maquillage ou de soin de la peau, y compris du cuir chevelu, aussi bien du visage que du corps humain, des lèvres ou des phanères des êtres humains, comme les cheveux, les cils, les sourcils ou les ongles, contenant un ester de masse moléculaire élevée, conférant à la composition des propriétés cosmétiques remarquables et en particulier de la brillance, du glissant, de la couvrance et de la tenue dans le temps.

La composition de l'invention peut en particulier constituer un produit de maquillage du corps, des lèvres ou des phanères d'êtres humains ayant des propriétés de soin et/ou de traitement. Elle constitue notamment un rouge à lèvres ou un brillant à lèvres, un fard à paupières, un produit pour tatouage, un mascara, un eye-liner, un vernis à ongles, un produit de bronzage artificiel de la peau, une crème de soin ou de protection éventuellement teintée, un produit de coloration ou de soin des cheveux.

Il existe de nombreuses compositions cosmétiques pour lesquelles les propriétés de brillance du film déposé, après application sur les matières kératiniques (peau, lèvres, phanères) sont très importantes. On peut citer par exemple les rouges à lèvres, les vernis à ongles ou encore certains produits capillaires.

Il est connu des formulateurs de produits cosmétiques que cette propriété de brillance est favorisée lorsque l'on utilise des huiles caractérisées par une viscosité et un indice de réfraction élevés et qui possèdent, en outre, de bonnes propriétés de dispersion des pigments ou des charges lorsque ces derniers sont présents dans la composition. Par huile, on entend un milieu non aqueux liquide à température ambiante (25°C) et pression atmosphérique (760 mm/Hg), non miscible à l'eau en toute proportion.

Dans cette optique le formulateur dispose de plusieurs types de matières premières, comme :
- des polymères huileux tels que les polybutènes, vendus notamment sous la référence Indopol H100, H300 et H1500 par la Société Amoco, qui ont une viscosité très élevée (typiquement supérieur à 28 194 cP (2 819 Pa.s) à 20°C mesurée avec un viscosimètre Brookfield "DV-II+" de type LV équipé d'une aiguille n°2 tournant à 0,5 tr/min) et un indice de réfraction élevé (typiquement supérieur à 1,49 à 20°C) mais qui présentent cependant l'inconvénient d'être à la fois très collants et de posséder des propriétés de dispersion des pigments relativement faibles, ce qui limite leur utilisation et leur concentration possible dans les produits ;
- des huiles d'origine végétale, comme l'huile de ricin très souvent utilisée dans la formulation des rouges à lèvres et qui se distingue par une viscosité moyenne (de l'ordre de 786 cP (78,6 Pa.s) à 20°C mesurée avec le viscosimètre mentionné précédemment tournant à 4 tr/min), un indice de réfraction de l'ordre de 1,475 à 25°C et de bonnes propriétés de dispersion des pigments, mais qui présente cependant l'inconvénient d'absorber de l'eau ce qui entraîne des problèmes de stabilité des produits en stick comme les rouges à lèvres (appelés exsudation), en particulier dans les pays où le taux d'humidité est très élevé, qui se caractérisent par des gouttelettes d'huile en surface du bâton de rouge à lèvres. L'huile de ricin est par ailleurs difficilement formulable dans un produit de soin, du fait de sa sensibilité à la chaleur et aux ultraviolets, entraînant sa dégradation partielle avec un dégagement d'odeur désagréable, difficile à camoufler même avec un parfum très odoriférant, ce qui en limite son utilisation dans des crèmes de soin mais aussi dans des fonds de teints fluides. Ces crèmes et font de teint sont généralement des émulsions.

Une solution pour limiter l'exsudation consisterait à éliminer la fonction alcool présente sur l'acide ricinoléïque entrant dans la constitution de l'huile de ricin, par exemple sous la forme d'un ester. On connaît ainsi le dérivé triacétylé de l'huile de ricin dont le nom selon l'association Cosmetic Toiletry and Fragance Association est glyceryl triacetyl ricinoleate, mais dont les propriétés physico-chimiques sont moins favorables que celles de l'huile de ricin vis-à-vis de la brillance notamment en ce qui concerne la viscosité (de l'ordre de 230 cP à 25 °C seulement).

L'invention a justement pour objet une composition de soin ou de maquillage de la peau et/ou des lèvres et/ou des phanères permettant de remédier à ces inconvénients. De façon surprenante, les inventeurs ont trouvé que l'utilisation d'un ou plusieurs esters issus de l'estérification totale ou partielle d'un composé aliphatique hydroxylé avec un acide aromatique permettait d'obtenir une composition brillante, homogène par suite d'une bonne dispersion des pigments ou des charges présents dans la composition, n'exsudant pas lorsqu'elle est sous forme de stick. Cette composition présente de bonnes propriétés d'étalement et de glissant et confère, en outre, au film déposé des propriétés de brillance, de bonne tenue dans le temps (non virage de couleur pendant au moins 3 heures, disparition du maquillage de façon homogène), de non collant et de non gras. Elle est, en outre, stable notamment plusieurs mois à température ambiante (25°C pendant plus d'un an), mais aussi à la chaleur (47°C pendant 2 mois) et aux ultraviolets, sans dégagement d'odeur désagréable.

De façon plus précise, l'invention a pour objet une composition cosmétique de soin ou de maquillage des matières kératiniques, sous forme coulée en stick, contenant au moins une charge particulaire, au moins une cire, et au moins un ester à groupement aromatique, liquide à température ambiante, choisi parmi le glyceryl monobenzoyl ricinoleate, le glyceryl mono-dibenzoyl ricinoleate, le glyceryl dibenzoyl ricinoleate, glyceryl tribenzoyl ricinoleate et leurs mélanges, la cire représentant 2 à 30% du poids total de la composition et la dureté de la composition étant comprise entre 100 et 250 g.

L'invention a aussi pour objet une composition cosmétique de soin ou de maquillage des matières kératiniques, sous forme coulée en stick, contenant au moins un agent émulsionnant, au moins une cire et au moins un ester à groupement aromatique, liquide à température ambiante, choisi parmi le glyceryl monobenzoyl ricinoleate, le glyceryl mono-dibenzoyl ricinoleate, le glyceryl dibenzoyl ricinoleate, glyceryl tribenzoyl ricinoleate et leurs mélanges, la cire représentant 2 à 30% du poids total de la composition et la dureté de la composition étant comprise entre 100 et 250 g.

La composition peut également comprendre au moins un ester résultant de l'estérification par un acide aromatique d'au moins un groupe hydroxyle pendant ou en bout de chaîne d'un composé aliphatique hydroxylé choisi parmi les acides aliphatiques hydroxylés et leurs esters.

Par composé aliphatique hydroxylé, on entend un acide hydroxy carboxylique aliphatique ou un ester d'acide hydroxy carboxylique aliphatique. L'acide (non estérifié) comporte notamment de 2 à 40 atomes de carbone, de préférence de 10 à 34 atomes de carbone et mieux de 12 à 28 atomes de carbone ; il comporte, en outre, de 1 à 20 groupes hydroxyle, de préférence de 1 à 10 groupes hydroxyle et mieux de 1 à 6 groupes hydroxyle, susceptible(s) d'être estérifié(s) par l'acide aromatique. Le composé hydroxylé sous forme d'ester résulte de l'estérification de la fonction -COOH d'un acide aliphatique hydroxy carboxylique par un alcool aliphatique qui peut comprendre de 1 à 40 atomes de carbone et mieux de 3 à 30 atomes de carbone. Cet alcool peut être un monoalcool ou un polyol. Les esters issus de la réaction de l'acide hydroxy carboxylique aliphatique avec un polyol peuvent être des esters partiellement ou totalement estérifiés.

De préférence le composé aliphatique hydroxylé est choisi parmi les esters issus d'acide hydroxy carboxylique aliphatique. Autrement dit, l'ester aromatique est un ester d'ester. Avantageusement, cet ester aromatique est un ester d'ester d'acide gras dont le reste d'acide gras comporte au moins 12 atomes de carbone. De préférence, le groupe hydroxyle engagé dans l'estérification par l'acide aromatique est porté sur la partie acide du composé hydroxylé.

La composition peut contenir un ou plusieurs esters aromatiques, liquides à température ambiante (25 °C) et pression atmosphérique (760 mm de Hg).

Lorsque le groupe hydroxyle du composé aliphatique hydroxylé engagé dans l'estérification avec l'acide aromatique est en bout de chaîne, ce groupe est en position α-w par rapport à la fonction -COOH de l'acide hydroxy carboxylique aliphatique.

De préférence, les esters aromatiques présentent une viscosité supérieure à 500 cP (50 Pa.s) à 20°C mais aussi à 23°C, de préférence allant de 900 à 10 000 cP (90 à 1 000 Pa.s) et mieux de 950 à 5 000 cP (95 à 500 Pa.s), mesurée en particulier avec un viscosimètre type Brookfield RV ou un viscosimètre type Brookfield "DV-II+" de type LV équipé d'une aiguille n° 1 tournant entre 0,5 et 10 tr/min, au bout de 10 minutes et/ou un indice de réfraction ≥ 1,48 et notamment allant de 1,48 à 1,55 (l'indice de réfraction étant défini pour la raie D du sodium).

L'acide aromatique peut être choisi parmi les acides carboxyliques suivants :
a) les mono acides, tels que l'acide benzoïque, l'acide phényl acétique, l'acide cinnamique, l'acide 3 phényl propanoïque, l'acide salicylique ;
b) les diacides tels que l'acide téréphtalique ;
c) les triacides tels que l'acide trimellitique ; et
d) les tétra-acides tels que l'acide pyromellitique.

De façon avantageuse, l'acide carboxylique aromatique est l'acide benzoïque.

Comme ester utilisable dans l'invention, on peut citer ceux résultant de l'estérification par au moins un acide aromatique de l'un au moins des acides carboxyliques hydroxylés aliphatiques suivants :
i) les monoacides aliphatiques mono hydroxylés linéaires saturés de formule: 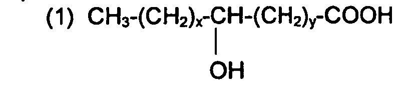 avec 0 ≤ x + y ≤ 37 comme l'acide lactique (x + y = 0) ; l'acide 12-hydroxy octadécanoïque (ou 12-hydroxy stéarique) de formule : 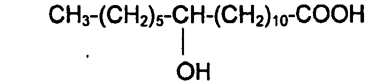 avec x = 5, y = 10 et x + y = 15 et l'acide α-hydroxy octadécanoïque 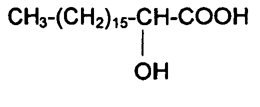 (avec x = 5 et y = 0) ou (2) HO-CH₂-(CH₂)ₓ-COOH avec 0 ≤ x ≤ 38 comme l'acide glycolique HO-CH₂-COOH (x = 0) ; ou l'acide junipérique (acide 16-hydroxy hexadécanoïque) de formule HO-CH₂-(CH₂)₁₄-COOH avec x = 14 ;
ii) les monoacides aliphatiques mono hydroxylés ramifiés saturés de formule : 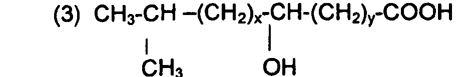 avec 0 ≤ x + y ≤ 35 comme l'acide 5-méthyl 2-hydroxy hexanoïque (acide leucinique) de formule : 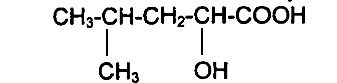 avec x = 1 y = 0 et x + y =1 ou (3') encore l'acide 2-éthyl 3-hydroxy caprylique de formule : 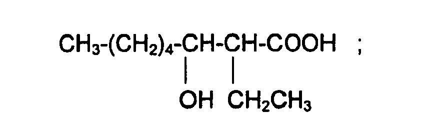
iii) Les monoacides aliphatiques mono hydroxylés insaturés de formule : 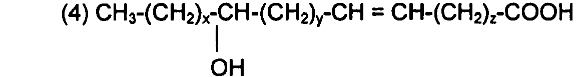 avec 0 ≤ x + y + z ≤ 35 comme l'acide 12-hydroxy (cis) 9-octadécénoïque (ou l'acide ricinoléïque) de formule : 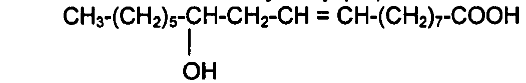 avec x = 5, y = 1, z = 7 et x + y + z = 13 ou 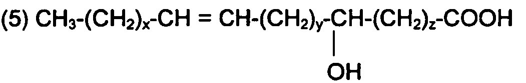 avec 0 ≤ x + y + z ≤ 35 comme l'acide 3-hydroxy 4-hexanoïque de formule avec x = 0, y = 0, z = 1 et x + y + z = 1 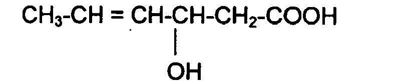 ou l'acide 2-hydroxy 15-tétracosènoïque (ou acide oxynervonique) de formule : 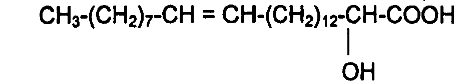 avec x = 7, y = 12, z = 0 et x + y + z = 17 ou (6) HOCH₂-(CH₂)ₓ-CH = CH-(CH₂)_{y}-COOH avec 0 ≤ x + y ≤ 36 comme l'acide 16-hydroxy 6-hexadécènoïque avec x = 8, y = 4 et x + y = 12 de formule HO-CH₂-(CH₂)₈-CH = CH-(CH₂)₄-COOH ;
iv) les monoacides aliphatiques poly hydroxylés saturés de formule : 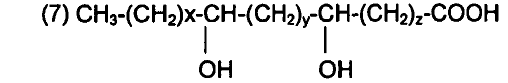 avec 0 ≤ x + y + z ≤ 36 comme l'acide 9, 10-dihydroxy octadécanoïque de formule 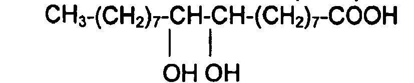 avec x = 7, y = 0, z = 7 et x + y + z = 14 comme l'acide 9, 12-dihydroxy octadécanoïque de formule 7 avec x = 5, y = 2, z = 7 et x + y + z = 14 ou l'acide 9, 10, 16-trihydroxy hexadécanoïque (acide aleuritique), l'acide 9, 10, 12 - trihydroxy octadécanoïque de formule 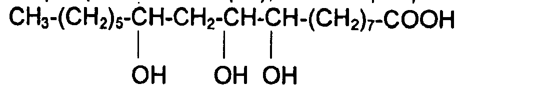 ou encore l'acide hexahydroxy octadécanoïque et l'acide octahydroxy octadécanoïque ;
v) les polyacides aliphatiques mono hydroxylés saturés de formule : 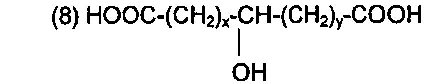 avec 0 ≤ x + y ≤ 37 comme l'acide malique, ou encore l'acide citrique ; et
vi) les polyacides aliphatiques poly hydroxylés saturés comme l'acide tartrique ;
et leurs mélanges.

Comme autre composé hydroxylé aliphatique utilisable dans l'invention, on peut citer ceux résultant de l'estérification par au moins un acide aromatique de l'un au moins des esters d'acides hydroxylés aliphatiques suivants :
vii) les esters de monoacides aliphatiques mono hydroxylés linéaires saturés tels que :
   - les esters de l'acide lactique comme le lactate d'isostéaryle, le lactate issu d'alcool en C₁₂-C₁₃, le lactate d'octyl dodécyle, le lactate d'oléyle, le lactate de myristyle ;
   - les esters de l'acide 12-hydroxy octadécanoïque (ou 12-hydroxy stéarique) comme l'hydroxy stéarate d'éthyl 2-hexyle, l'hydroxy stéarate d'octyl docécyle, l'hydroxy stéarate d'isostéaryle, l'hydroxy stéarate d'isodécyle, le trihydroxy stéarate de glycéryle (ou huile de ricin hydrogénée), l'hexahydroxy stéarate de dipentaérythrityle ;
viii) les esters de monoacides aliphatiques mono hydroxylés insaturés tels que les esters de l'acide ricinoléique (ou acide 12-hydroxy (cis) 9-octadécénoïque) comme le ricinoléate de butyle, le ricinoléate d'octyl dodécyle, le ricinoléate de cétyle, le triricinoléate de glycéryle (huile de ricin) ;
ix) les esters de polyacides aliphatiques mono hydroxylés saturés tels que le malate de diisostéaryle, le citrate de triisostéaryle, le citrate de trioctyl dodécyle ;
x) les esters de polyacides aliphatiques poly hydroxylés saturés comme le tartrate issu de la réaction avec 2 alcools en C₁₂-C₁₃ ramifiés ;
et leurs mélanges.

Comme composé hydroxylé sous forme d'ester, utilisable dans l'invention et résultant de l'estérification d'un polyol, on peut citer de façon générale :
xi) les esters partiels ou totaux de polyol en C₂ à C₁₆ ayant réagit avec un acide aliphatique hydroxylé comme notamment les triglycérides, les esters du pentaérythritol, du néopentyl glycol, du dipentaérythritol, du polyglycérol, les esters du sorbitol ;
et leurs mélanges.

De préférence, les esters aromatiques sont choisis parmi les esters d'ester d'acides gras aliphatiques dont le reste d'acide gras comporte au moins 12 atomes de carbone. En particulier, le composé hydroxylé est choisi parmi les esters de l'acide ricinoléïque, les esters de l'acide 12-hydroxy stéarique, les esters de l'acide lactique, les esters de l'acide hydroxy 14-eicosènoïque et leurs mélanges.

Le glyceryl monobenzoyl ricinoleate peut être l'ester résultant de la réaction d'estérification de l'huile de ricin avec l'acide benzoïque dans les proportions de 1 pour 1 (1/1) par exemple commercialisé par la société Finetex sous la référence Finsolv BCO-110.
Le glyceryl mono -dibenzoyl ricinoleate peut être le composé résultant de la réaction de l'huile de ricin avec l'acide benzoïque dans les proportions de 1 pour 1,5 (1/1,5) par exemple commercialisé par la société Finetex sous la référence Finsolv BCO-115,.
Le glyceryl dibenzoyl ricinoleate peut être le composé résultant de la réaction d'estérification de l'huile de ricin avec l'acide benzoïque dans les proportions de 1 pour 2 (1/2) par exemple commercialisé par la société Finetex sous la référence Finsolv BCO-120.
Le glyceryl tribenzoyl ricinoleate peut être le composé résultant de la réaction d'estérification de l'huile de ricin avec l'acide benzoïque dans les proportions de 1 pour 3 (1/3) par exemple commercialisé par la société Finetex sous la référence Finsolv BCO-130.

L'acide ricinoléique représente de 80 à 92 % de l'huile de ricin. Ainsi son estérification conduit majoritairement (80 à 92 %) à l'ester de l'ester de l'acide ricinoléïque.

Les esters aromatiques de l'huile de ricin ont des viscosités et des indices de réfractions supérieurs à ceux de l'huile de ricin :

| | Huile de ricin | Finsolv BCO110 | Finsolv BCO115 | Finsolv BCO120 | Finsolv BCO 130 |
|---|---|---|---|---|---|
| Viscosité à 20°C (cP)** | 786 (78,6 Pa.s) (4)* | 1008 (100,8 Pa.s) (4)* | 2699 (269,9 Pa.s) (1)* | 1045 (104,5 Pa.s) (4)* | 918 (91,8 Pa.s) (4)* |
| Viscosité à 23°C (cP)*** | 844 (84,4 Pa.s) (10) * | 971 (97,1 Pa.s) (10) * | - | 1036 (103,6 Pa.s) (5) * | 1090 (109 Pa.s) (5) * |
| Indice de réfraction à 25°C | 1,475 | 1,488 | 1,494 | 1,496 | 1,498 |

| | | | | | |
|---|---|---|---|---|---|
| (* vitesse de rotation en tr/min). (** La viscosité à 20°C est mesurée au viscosimètre Brookfield "DV-II+" type LV avec un mobile n°1). (*** La viscosité à 23°C est mesurée avec un viscosimètre Brookfield RV, équipé d'une aiguille n°1). | | | | | |

Les viscosités et indices de réfraction des esters aromatiques sont, dans chaque cas, supérieurs à ceux de l'huile de ricin.

Les propriétés de dispersion des particules solides, comme par exemple les pigments ou les charges, des esters aromatiques de l'invention sont supérieures à celles de l'huile de ricin et en particulier vis-à-vis de l'oxyde de fer brun (voir exemples).

Les esters aromatiques de l'invention absorbent tous beaucoup moins d'eau que l'huile de ricin, en particulier le Finsolv BCO-115, le Finsolv BCO-120 et le Finsolv BCO-130 (voir exemples). Ils ont donc moins tendance à provoquer des problèmes de stabilité plus spécialement dans les pays où le taux d'humidité est élevé.

Enfin leurs propriétés cosmétiques sont comparables à celles de l'huile de ricin et supérieures à celles du polybutène (Indopol H 1500) sur plusieurs critères en particulier le collant pour une brillance comparable (voir exemples).

L'ester aromatique de la composition de l'invention peut être fabriqué selon le procédé décrit dans le document US-A-5 959 130.

L'ester aromatique de la composition de l'invention peut représenter de 0,1 à 99,9 % du poids total de la composition, de préférence de 1 à 99 % et mieux de 5 à 90 %, et de façon générale, être présent en une quantité suffisante pour conférer à la composition des propriétés de non gras, de non collant, de glissant, de brillance, de couvrance, de stabilité et/ou de tenue dans le temps.

Les applications sont multiples et concernent l'ensemble des produits cosmétiques ou dermatologiques colorés ou non et plus particulièrement les rouges à lèvres.

La composition de l'invention se présente sous la forme coulée en stick.

La composition de l'invention comprend avantageusement une matière colorante qui peut être choisie parmi les colorants solubles ou dispersibles dans la composition, les pigments, les nacres et leurs mélanges. Les colorants sont de préférence des colorants liposolubles, bien que les colorants hydrosolubles puissent être utilisés. Cette matière colorante peut représenter de 0,001 à 98 %, de préférence de 0,5 à 85% et mieux de 1 à 60 % du poids total de la composition. Les teneurs en matière colorante supérieures à 50 % sont principalement destinées à une composition sous forme de poudre. Dans, ce cas, l'ester aromatique peut constituer tout ou partie du liant de poudre.

Pour une composition sous forme coulée telle que les fonds de teint, les rouges à lèvres ou les produits de maquillage du corps, on utilise en général de 0,5 à 50 % de matière colorante, de préférence de 2 à 40 % et mieux de 5 à 30%, par rapport au poids total de la composition.

Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

De préférence, la composition de l'invention, comprend une charge particulaire pouvant représenter de 0 à 50 % du poids total de la composition, de préférence de 0,01 à 40 % et mieux de 0,05 à 30 %, et qui peut comprendre des pigments et/ou des nacres et/ou des charges habituellement utilisés dans les compositions cosmétiques ou dermatologiques.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré, les pigments goniochromatiques et par exemple les pigments multicouches interférentiels.

Les charges peuvent être présentes à raison de 0 à 35 % du poids total de la composition, de préférence 0,5 à 15 %. On peut notamment citer le talc, le mica, le kaolin, les poudres de Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Corning), le Polypore® L 200 (Chemdal Corporation) et les microbilles de résine de silicone (Tospearl ® de Toshiba, par exemple).

La composition contient

au moins une cire. Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, et présentant à l'état solide une organisation cristalline anisotrope. La taille des cristaux est telle que les cristaux diffractent et/ou diffusent la lumière, conférant à la composition un aspect trouble, plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinées à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 30°C et mieux supérieure à 45°C.

Comme cire utilisable dans l'invention, on peut citer celles généralement utilisées dans les domaines cosmétique et dermatologique : elles sont notamment d'origine naturelle éventuellement modifiée comme la lanoline, la lanoline oxypropylénée ou acétylée, la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ou l'huile de ricin hydrogénée ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 30°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone ; et leurs mélanges.

Du fait des bonnes propriétés de dispersion de la phase particulaire dans la composition de l'invention, la cristallisation de la ou des cires dans la composition est favorisée. Il s'ensuit une intégrité et un homogénéité du réseau cristallin des cires renforçant les propriétés mécaniques de la composition, ce qui est particulièrement intéressant lorsque celle-ci est sous forme de stick. En particulier, la dureté du stick est accrue avec les esters aromatiques de la composition selon l'invention, par rapport à une composition de l'art antérieur sans ces esters.

La dureté peut être mesurée par la méthode dite "du fil à couper le beurre", qui consiste à couper un bâton de rouge à lèvres de 12,7 mm et à mesurer la dureté à 20°C, au moyen d'un dynamomètre DFGHS 2 de la société Indelco-Chatillon se déplaçant à une vitesse de 100mm/minute. Elle est exprimée comme la force de cisaillement (exprimée en gramme) nécessaire pour couper un stick dans ces conditions. Selon cette méthode la dureté d'une composition en stick selon l'invention va notamment de 100 à 250 g et par exemple de 150 à 230 g.

Les gommes utilisables dans l'invention se présentent généralement sous forme solubilisée dans une huile, les polymères sont solides à température ambiante et les résines peuvent être liquides ou solides à température ambiante.

La nature et la quantité des gommes, corps pâteux ou cires sont fonction des propriétés mécaniques et des textures recherchées. A titre indicatif, la composition peut contenir de 0,01 à 50 % en poids de cires, par rapport au poids total de la composition, de préférence de 2 à 40 %, et mieux de 5 à 30 %.

Les huiles peuvent être des huiles hydrocarbonées et/ou siliconées et/ou fluorées. Ces huiles peuvent être d'origine animale, végétale, minérale ou synthétique. "Par huile hydrocarbonée", on entend une huile comportant principalement des atomes de carbone et d'hydrogène et éventuellement une ou plusieurs fonctions choisies parmi les fonctions hydroxyle, ester, éther, carboxylique. A titre d'exemple d'huile utilisable dans l'invention, on peut citer :
- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, le polyisobutène hydrogéné tel que le Parleam ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec R₁ + R₂ ≥ 10 comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates,
- leurs mélanges.

Les huiles additionnelles peuvent représenter de 0 à 90 % du poids total de la composition, de préférence de 0,05 à 60 % et mieux de 1 à 35 %.

La composition de l'invention peut comprendre, en outre, tout additif usuellement utilisé dans le domaine concerné, tel que l'eau, des antioxydants, des conservateurs, des neutralisants, des gélifiants lipophiles ou de corps gras liquides, des gélifiants de phase aqueuse, des dispersants, des actifs cosmétiques ou dermatologiques. Ces additifs, à l'exception de l'eau qui peut représenter de 0 à 70 % et par exemple de 1 à 50 et mieux ce 1 à 10 % du poids total de la composition, peuvent être présents dans la composition à raison de 0 à 20% du poids total de la composition et mieux de 0 à 10%.

Dans le cas d'une émulsion ou dispersion d'une phase aqueuse dans une phase grasse, la composition contient avantageusement un émulsionnant. Les esters aromatiques, du fait de leur bonne propriété de dispersion en particulier des particules solides (ou phase particulaire) facilitent l'obtention d'émulsions stables dans le temps, ne démixant pas.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut se présenter sous la forme d'une composition, colorée ou non, dermatologique ou de soin de la peau, sous forme d'une composition de protection solaire ou de démaquillage ou encore sous forme d'une composition hygiénique. Elle contient notamment des actifs cosmétiques ou dermatologiques. Elle peut alors être utilisée comme base de soin ou de traitement pour la peau comme les mains ou le visage ou pour les lèvres (baumes à lèvres, protégeant les lèvres du froid et/ou du soleil et/ou du vent) ou encore comme déodorant. Comme actif cosmétique utilisable dans l'invention, on peut citer les vitamines A, E, C, B₃, les provitamines comme le D-panthénol, les actifs apaisants comme l'a-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

La composition de l'invention peut également se présenter sous la forme d'un produit de maquillage de la peau, en particulier du visage comme un fond de teint, un blush, un fard comme un produit de tatouage semi-permanent ou de maquillage des lèvres comme un rouge ou un brillant à lèvres, présentant éventuellement des propriétés de soin ou de traitement, un produit de maquillage des phanères comme par exemple un vernis à ongles, un mascara, un eyeliner, un produit de coloration ou de soin des cheveux.

Bien entendu la composition de l'invention doit être physiologiquement acceptable (cosmétiquement ou dermatologiquement acceptable), à savoir non toxique et susceptible d'être appliquée sur la peau, les phanères ou les lèvres d'êtres humains.

La composition selon l'invention peut être fabriquée par les procédés connus, généralement utilisés dans le domaine cosmétique ou dermatologique.

L'invention a encore pour objet un procédé cosmétique de soin ou de maquillage de la peau, des lèvres ou des phanères des êtres humains, comprenant l'application sur la peau, les lèvres ou les phanères de la composition cosmétique telle que définie ci-dessus.

L'invention est illustrée plus en détail dans les exemples suivants. Les quantités sont données en pourcentage massique. Dans la suite de la description, les duretés indiquées ont toutes été mesurées selon la méthode "du fil à couper le beurre" indiqué précédemment.

### Exemples 1 à 3 : rouge à lèvres en stick :

Les inventeurs ont reproduit l'exemple n° 3 du document de L'Oréal FR-A- 2 745 580 pour lequel ils ont successivement substitué le polybutène commercialisé sous la référence Indopol H1500 par la société Amoco (noté exemple 1 comparatif) par :
- l'huile de ricin (exemple 2 comparatif),
- le glyceryl dibenzoyl ricinoleate (Finsolv BCO-120), (exemple 3).

Les compositions sont portées dans le tableau (I).

**TABLEAU (I)**

| | Ex N° 1 (comparatif) | Ex N° 2 (comparatif) | Ex N°3 (invention) |
|---|---|---|---|
| Polybutène | 15,00 | - | - |
| Huile de ricin | - | 15,00 | - |
| Glyceryl dibenzoyl ricinoleate | - | - | 15,00 |
| Benzoate d'alkyles en C₁₂-C₁₅ | 20,00 | 20,00 | 20,00 |
| Trimellitate de tridécyle | 15,00 | 15,00 | 15,00 |
| Phenyltrimethicone (DC556) | 5,00 | 5,00 | 5,00 |
| Malate de diisostéaryle | 7,00 | 7,00 | 7,00 |
| Lanoline | 13,00 | 13,00 | 13,00 |
| Conservateur, antioxydants | 0,10 | 0,10 | 0,10 |
| Cire de polyéthylène (Mw= 500) | 4,00 | 4,00 | 4,00 |
| Cire de Candelilla | 7,00 | 7,00 | 7,00 |
| Cire de Carnauba | 3,90 | 3,90 | 3,90 |
| Pigments | 10,00 | 10,00 | 10,00 |
| | 100,00 % massique | 100,00 % massique | 100,00 % massique |

### Mode opératoire

Les pigments sont broyés dans la phase huileuse au broyeur tricylindre. On ajoute ensuite les autres constituants et l'on chauffe à 100°C jusqu'à homogénéisation et fusion complète des cires. Puis on coule le mélange dans un moule adéquat pour obtenir des sticks qui sont ensuite conditionnés.

### Evaluation sensorielle

Les exemples 1 à 3 ont été comparés deux à deux par application par demi-lèvres sur un panel d'expertes.

Ainsi, la composition 3 selon l'invention a été jugée plus glissante, plus douce, plus couvrante, moins collante, plus agréable à porter et de meilleure tenue que la composition 1 et, plus douce, de maquillage plus homogène, plus couvrant, moins collant, plus agréable, plus brillant et de meilleure tenue dans le temps que la composition 2.

De plus, la composition 3 présente une dureté de stick de 223 g, supérieure à celle des compositions 1 et 2 respectivement de 206 g et 200 g, pour une même quantité d'ingrédients. Elle est donc plus résistante d'un point de vue mécanique. En outre, pour obtenir une dureté de stick identique à celle d'un stick de l'art antérieur, on peut diminuer la quantité de cire, ce qui améliore alors la brillance du stick.

### Exemples 4 et 5 : dispersion des pigments

Les inventeurs ont comparé les propriétés dispersantes des esters aromatiques de la composition de l'invention avec celles de l'huile de ricin vis-à-vis de l'oxyde de fer brun vendu sous la référence Cosmetic Grade Red Iron Oxide BC-7051 par la société Warner-Jenkinson.

### Mode opératoire

On prépare, dans un premier temps, un mélange constitué de 16,67 % d'oxyde de fer brun et 83,33 % massique d'huile (2 g de pigment pour 10 g d'huile) que l'on chauffe à 70°C en mélangeant manuellement de manière à réaliser le mouillage du pigment.

On broie ensuite ce mélange au broyeur tricylindre en effectuant trois passages. Puis on dilue ce broyat en ajoutant de l'huile (88 g d'huile pour un mélange de 2 g de pigment et 10 g d'huile au départ) de manière à obtenir une dispersion de pigment contenant :

| | |
|---|---|
| 2,00 % | d'oxyde de fer brun |
| 98,00 % | d'huile |
| 100,00 % | massique |

On prépare séparément un support constitué d'un papier filtre « sans cendre » d'une porosité de 25 µm et d'un diamètre de 90 mm, commercialisé par la société Prolabo que l'on imprègne dans un cristallisoir avec l'huile testée, préalablement chauffée à 70°C.

Après imprégnation on élimine l'huile excédentaire de manière à obtenir un support de masse comprise entre 1,02 g et 1,18 g.

On dépose ensuite au centre du support 0,05 g de la dispersion et on laisse opérer le mouvement d'imprégnation et de diffusion des pigments dans le support pendant une période de 24 heures à température ambiante (25°C).

On effectue la mesure du diamètre de l'auréole pigmentaire obtenu à l'aide d'une règle millimétrique. Les résultats sont donnés dans le tableau (II), ci-après.

**TABLEAU (II)**

| **Huile** | **Diamètre (mm) de l'auréole pigmentée** |
|---|---|
| Glyceryl monobenzoyl ricinoleate (Finsolv BCO 110) | 41 |
| Glyceryl tribenzoyl ricinoleate (Finsolv BCO 130) | 36 |
| Glyceryl mono -dibenzoyl ricinoleate (Finsolv BCO 115) | 40 |
| Glyceryl dibenzoyl ricinoleate (Finsolv BCO 120) | 35 |
| Huile de ricin | 27 |

Ces résultats montrent que les esters de l'invention ont des propriétés de dispersion vis-à-vis de l'oxyde de fer brun supérieures à celles de l'huile de ricin. Plus le diamètre est élevé, meilleure est la dispersion.

### Exemples 6 et 7 : absorption d'eau

Les inventeurs ont comparé les propriétés d'absorption d'eau des esters aromatiques de la composition de l'invention avec celles d'une composition contenant de l'huile de ricin à partir d'un stick de formule suivante :

| | |
|---|---|
| Octacosanyl stéarate | 9,00 % |
| Huile | 91,00 % |
| | 100,00 % massique |

### Mode opératoire

Les sticks ont été préparés de manière habituelle en chauffant le mélange à 100°C de manière à fondre la cire puis coulage à cette température dans un moule adéquat.

Les sticks sont ensuite entreposés dans une étuve à 25°C et 90 % d'humidité relative.

On mesure alors l'augmentation de leur masse au cours du temps, augmentation qui correspond à l'absorption d'eau du stick. Les résultats sont portés sur le graphe annexé qui donne la différence de masse Delta M(g) en fonction du temps (j). Ce graphe a été établi à partir des données du tableau(lll).

**TABLEAU (III)**

| Temps (Jours) | Delta M Huile de Ricin (g) | Delta M Glyceryl monobenzoyl ricinoleate (g) | Delta M Glyceryl dibenzoyl ricinoleate (g) | Delta M Glyceryl tribenzoyl ricinoleate (g) |
|---|---|---|---|---|
| 0 | 0 | 0 | 0 | 0 |
| 1 | 0,0052 | 0,0033 | 0,0026 | 0,0025 |
| 2 | 0,0069 | 0,0049 | 0,0037 | 0,0037 |
| 3 | 0,0083 | 0,0059 | 0,0046 | 0,0048 |
| 6 | 0,0123 | 0,0092 | 0,0073 | 0,0078 |
| 7 | 0,013 | 0,0097 | 0,0073 | 0,0079 |
| 8 | 0,0136 | 0,01 | 0,0076 | 0,0082 |
| 9 | 0,0141 | 0,0105 | 0,0079 | 0,0085 |
| 14 | 0,0161 | 0,0121 | 0,0087 | 0,0095 |
| 15 | 0,0171 | 0,0131 | 0,0095 | 0,0104 |
| 16 | 0,0175 | 0,0134 | 0,0097 | 0,0105 |
| 17 | 0,0179 | 0,0137 | 0,0098 | 0,0107 |
| 20 | 0,0187 | 0,0143 | 0,0101 | 0,011 |
| 22 | 0,019 | 0,0146 | 0,0101 | 0,0109 |
| 24 | 0,019 | 0,0144 | 0,0097 | 0,0104 |

De ce graphe, on constate que les sticks de l'invention absorbent moins d'eau que ceux contenant l'huile de ricin.

### Exemples 8 à 10 : rouge à lèvres

Les exemples de compositions sont portés dans le tableau (IV) ci-après.

**TABLEAU (IV)**

| | Exemple 8 (comparatif) | Exemple 9 (comparatif) | Exemple 10 (invention) |
|---|---|---|---|
| Polybutène | 15,58 | - | - |
| Huile de ricin | - | 15,58 | - |
| Glyceryl dibenzoyl ricinoleate (Finsolv BCO-120) | - | - | 15,58 |
| Benzoate d'alkyles en C₁₂-C₁₅ | 20,78 | 20,78 | 20,78 |
| Trimellitate de tridécyle | 15,58 | 15,58 | 15,58 |
| Phenyltrimethicone DC556) | 5,19 | 5,19 | 5,19 |
| Malate de diisostéaryle | 7,27 | 7,27 | 7,27 |
| Lanoline | 13,50 | 13,50 | 13,50 |
| Conservateur, antioxydants | 0,10 | 0,10 | 0,10 |
| Cire de polyéthylène (Mw : 500) | 3,22 | 3,22 | 3,22 |
| Cire de Candelilla | 5,64 | 5,64 | 5,64 |
| Cire de Carnauba | 3,14 | 3,14 | 3,14 |
| Pigments | 10,00 | 10,00 | 10,00 |
| | 100 % massique | 100 % massique | 100 % massique |

Les sticks 8 à 10 sont fabriqués conformément aux exemples 1 à 3. La dureté des sticks obtenus pour les exemples 8, 9, 10 est respectivement de 145 g, 137 g et 188 g. Dans ces exemples, on peut surtout noter une diminution du taux de cire par rapport aux exemples 1 à 3. Avec la composition 10 de l'invention, on obtient une dureté tout à fait acceptable pour un rouge à lèvres avec moins de 15 % de cire. Il présente une brillance améliorée par rapport aux compositions 8 et 9 selon l'art antérieur.

### Evaluation sensorielle

Ces trois compositions ont été comparées deux par deux par application en demi-lèvres sur un panel de sept expertes. Les notations sur les différents critères sont comprises entre 0 (pas) et 10 (très) inclus. Les résultats sont portés dans les tableaux (V) et (VI), ci-après.

### 1) Comparaison des exemples N° 8 et 10

**TABLEAU (V)**

| | **Ex. N° 8 (Comparatif)** | **Ex. N° 10 (Invention)** |
|---|---|---|
| **Critères évalués** | **Valeur Ecart type** | **Valeur Ecart type** |
| • **A l'application du Stick sur les lèvres** | | |
| Glissant | 3,6 ± 1,1 | 6,4 ± 0,8 |
| Douceur | 5,0 ± 0,8 | 6,7 ± 2,0 |
| Collant | 4,1 ± 3,1 | 3,0 ± 1,4 |
| Homogénéité | 7,6 ± 0,8 | 7,6 ± 1,1 |
| Brillance | 7,0 ± 1,2 | 6,9 ± 1,2 |
| Couvrance * | 7,0 ± 1,0 | 7,3 ± 1,0 |

| • **Après application** | | |
|---|---|---|
| Collant | 4,9 ± 2,9 | 3,9 ± 2,2 |
| Brillance | 6,6 ± 1,0 | 6,7 ± 1,1 |
| Agrément d'application | 5,0 ± 0,8 | 7,4 ± 1,0 |
| Qualité du produit | 5,3 ± 0,8 | 7,1 ± 0,9 |

| • **Après 1 heure d'application** | | |
|---|---|---|
| Confort | 6.1 ± 1,2 | 7,1 ± 1,6 |
| Collant | 3,9 ± 2,2 | 2,9 ± 2,1 |
| Tenue | 5,7 ± 1,1 | 6,3 ± 0,8 |
| Brillance | 6,1 ± 1,4 | 5,7 ± 1,4 |
| Opinion générale | 4,9 ± 0,7 | 5,9 ± 1,4 |

| | | |
|---|---|---|
| (* la couvrance est l'aptitude d'une composition de maquillage pour recouvrir le support sur lequel est appliquée ladite composition, notamment pour en cacher les imperfections). | | |

La composition 10 de l'invention est significativement plus glissante à l'application que la composition 8 ; son agrément à l'application et la qualité (propriétés cosmétiques) du produit sont aussi significativement supérieurs à ceux de la composition 8.

La composition de l'invention a tendance à être moins collante et plus confortable que celle de l'exemple 8 ; son opinion générale est meilleure pour une brillance comparable.

### 2) Comparaison des exemples N° 9 et 10

**TABLEAU (VI)**

| | **Ex. N° 9 (Comparatif)** | **Ex. N° 10 (Invention)** |
|---|---|---|
| **Critères évalués** | **Valeur Ecart type** | **Valeur Ecart type** |
| **• A l'application du stick sur les lèvres** | | |
| Glissant | 6,1 ± 1,1 | 6,0 ± 1,0 |
| Douceur | 6,6 ± 1,3 | 7,0 ± 0,8 |
| Collant | 2,0 ± 1,4 | 2,1 ± 1,8 |
| Homogénéité | 6,4 ± 1,1 | 6,9 ± 1,1 |
| Brillance | 6,4 ± 1,0 | 6,4 ± 1,0 |
| Couvrance | 6,6 ± 1,0 | 6,9 ± 0,7 |

| • **Après application** | | |
|---|---|---|
| Collant | 2,4 ± 1,5 | 3,0 ± 1,7 |
| Brillance | 6,6 ± 1,0 | 6,6 ± 1,0 |
| Agrément d'application | 6,6 ± 1,0 | 7,1 ± 0,7 |
| Qualité du produit | 6,3 ± 0,5 | 7,0 ± 0,8 |

| • **Après 1 heure d'application** | | |
|---|---|---|
| Confort | 6,0 ± 1,3 | 5,4 ± 1,3 |
| Collant | 3,1 ± 2,2 | 3,3 ± 2,2 |
| Tenue | 6,3 ± 1,1 | 6,6 ± 1 ,0 |
| Brillance | 5,6 ± 1,1 | 5,9 ± 1,3 |
| Opinion générale | 5,4 ± 1,0 | 5,4 ± 1,4 |

La composition n° 10 selon l'invention a tendance à être d'une qualité supérieure, après application, pour une brillance comparable tout au cours du test, à celle de l'exemple 9.

### Exemple 11 : rouge à lèvres (Invention)

| | **% massique** |
|---|---|
| Glyceryl mono -dibenzoyl ricinoleate (Finsolv BCO 115) | 15,58 |
| Benzoate d'alkyles en C₁₂-C₁₅ | 20,78 |
| Trimellitate de tridécyle | 15,58 |
| Phenyltrimethicone (DC556) | 5,19 |
| Malate de diisostéaryle | 7,27 |
| Lanoline | 13,50 |
| Conservateur, antioxydants | 0,10 |
| Cire de polyéthylène (Mw = 500) | 3,22 |
| Cire de candelilla | 5,64 |
| Cire de Carnauba | 3,14 |
| Pigments | 10,00 |
| | 100 % massique |

Ce stick 11 est préparé conformément aux exemples 1 à 3. Il présente une dureté de 160g. Il permet le dépôt d'un maquillage sur les lèvres non gras, non collant, doux, brillant, de bonne tenue, couvrant, homogène et agréable à porter toute la journée.

### Absorption de l'eau

Le stick de l'exemple 10 et celui de l'exemple 11, conditionnés dans un étui de rouge à lèvres ont été entreposés (sans capot et bâton ou raisin non sorti) dans une étuve à 30°C et 70 % d'humidité relative. On a alors mesuré leur augmentation de masse au cours du temps. Les résultats sont portés dans le tableau (VII).

**TABLEAU (Vll)**

| **ABSORPTION D'EAU** | | |
|---|---|---|
| Temps (Jours) | Delta / Masse (g) Finsolv BCO-120 | Delta / Masse (g) Finsolv BCO-115 |
| 0 | 0,0000 | 0,0000 |
| 1 | 0,0115 | 0,0109 |
| 2 | 0,0079 | 0,0054 |
| 5 | 0,0155 | 0,0178 |
| 6 | 0,0196 | 0,0169 |
| 7 | 0,0204 | 0,0187 |
| 8 | 0,0199 | 0,0173 |
| 9 | 0,0187 | 0,0193 |
| 12 | 0,0246 | 0,0224 |
| 13 | 0,0253 | 0,0226 |
| 14 | 0,0252 | 0,0220 |
| 15 | 0,0247 | 0,0233 |

On constate d'après ce tableau (VII) que les sticks selon l'invention absorbent peu d'eau. On constate aussi que le stick selon l'exemple 11 absorbe encore moins d'eau que celui de l'exemple 10.

### Exemples 12 et 13 : dispersion de pigments

**TABLEAU (VIII)**

| Pigment | Huile diamètre (mm) de l'auréole pigmentée | |
|---|---|---|
| | Finsolv BCO 115 | Huile de ricin |
| Oxyde de fer brun | 40 | 27 |
| Oxyde de fer rouge | 40 | 26 |
| Oxyde de fer jaune | 39 | 24 |
| Oxyde de fer noir | 23 | 22 |
| Dioxyde de titane (vendu par Kerr Mac Gee Chemical Corp. sous la ref. Tronox CR837 CTFA) | 39 | 29 |
| FD & C Yellow n°5 Al lake | 38 | 29 |
| FD & C Yellow n°6 Al lake | 41 | 32 |
| D & C Red 7 | 37 | 29 |
| D & C Red 21 Al lake | 42 | 34 |
| D & C Red 27 Al lake | 38 | 30 |
| FD & C Blue N°1 Al lake | 33 | 29 |

Du tableau (VIII), il ressort que dans tous les cas, le diamètre de l'auréole, lié à la qualité de dispersion, est très supérieur avec le glyceryl mono -dibenzoylricinoleate (Finsolv BCO115) par rapport à l'huile de ricin. 1

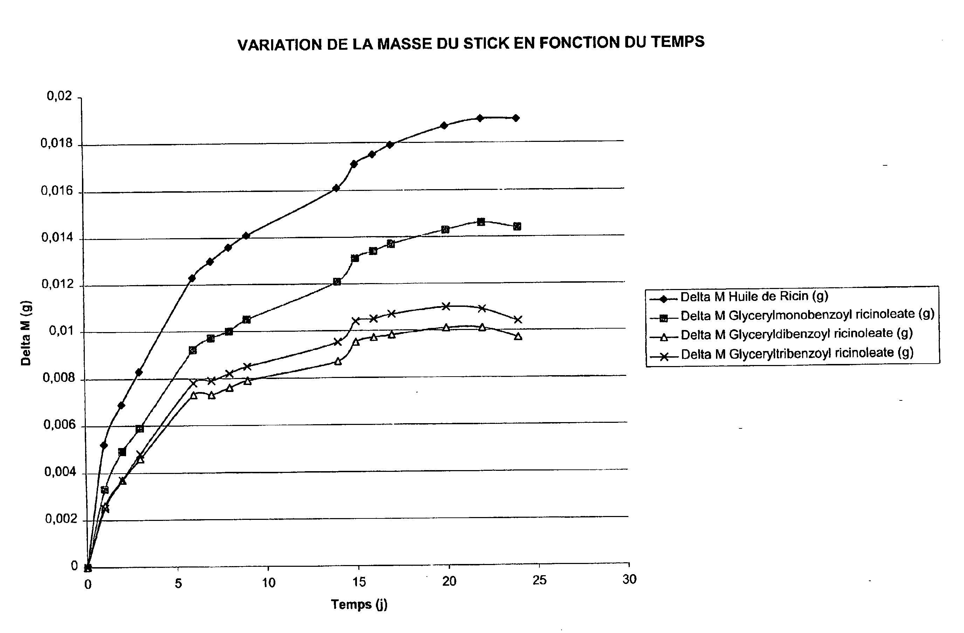

## Revendications

1. Composition cosmétique de soin ou de maquillage des matières kératiniques, sous forme coulée en stick, contenant :
- au moins une charge particulaire,
- au moins une cire, et
- au moins un ester à groupement aromatique, liquide à température ambiante, choisi parmi le glyceryl monobenzoyl ricinoleate, le glyceryl mono-dibenzoyl ricinoleate, le glyceryl dibenzoyl ricinoleate, glyceryl tribenzoyl ricinoleate et leurs mélanges,
ladite cire représentant 2 à 30% du poids total de la composition et la dureté de ladite composition étant comprise entre 100 et 250 g.

2. Composition cosmétique de soin ou de maquillage des matières kératiniques, sous forme coulée en stick, contenant :
- au moins un agent émulsionnant,
- au moins une cire, et
- au moins un ester à groupement aromatique, liquide à température ambiante, choisi parmi le glyceryl monobenzoyl ricinoleate, le glyceryl mono-dibenzoyl ricinoleate, le glyceryl dibenzoyl ricinoleate, glyceryl tribenzoyl ricinoleate et leurs mélanges,
ladite cire représentant 2 à 30% du poids total de la composition et la dureté de ladite composition étant comprise entre 100 et 250 g.

3. Composition selon l'une des revendications précédentes, **caractérisée en ce que** l'ester aromatique est présent en une quantité allant de 0,1 à 99,9 %, de préférence de 1 à 99 % et mieux de 5 à 90% du poids total de la composition.

4. Composition selon l'une des revendications précédentes, **caractérisée en ce qu'**elle contient, en outre, au moins un corps gras additionnel choisi parmi les corps gras liquides à température ambiante différents dudit ester aromatique, les corps gras pâteux, les gommes, les résines, les polymères lipophiles et leurs mélanges.

5. Composition selon l'une des revendications précédentes, **caractérisée en ce qu'**elle contient au moins une matière colorante.

6. Composition selon la revendication précédente, **caractérisée en ce que** la matière colorante est choisie parmi les colorants solubles ou dispersibles dans la composition, les pigments, les nacres et leurs mélanges.

7. Composition selon l'une des revendications précédentes, **caractérisée en ce qu'**elle contient, en outre, un aditif choisi parmi les antioxydants, les actifs cosmétiques ou dermatologiques, les conservateurs, les gélifiants de corps gras liquides, les dispersants et leurs mélanges.

8. Composition selon l'une des revendications précédentes, **caractérisée en ce qu'**elle contient un actif cosmétique choisi parmi les vitamines A, E, C, B₃, les provitamines comme le D-panthénol, les actifs apaisants comme l'α-bisabolol l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

9. Composition selon l'une des revendications précédentes, **caractérisée en ce qu'**elle se présente sous forme d'un rouge à lèvres.

10. Procédé cosmétique de soin ou de maquillage de la peau ou des lèvres des êtres humains, comprenant l'application sur la peau ou les lèvres de la composition cosmétique selon l'une des revendications précédentes.

## Claims

1. Cosmetic composition for caring for or for making up keratinous substances, in the form cast as a stick, comprising:
- at least one particulate filler,
- at least one wax, and
- at least one ester comprising an aromatic group which is liquid at room temperature chosen from glyceryl monobenzoyl ricinoleate, glyceryl mono-dibenzoyl ricinoleate, glyceryl dibenzoyl ricinoleate, glyceryl tribenzoyl ricinoleate and their mixtures,
the said wax representing 2 to 30% of the total weight of the composition and the hardness of the said composition being between 100 and 250 g.

2. Cosmetic composition for caring for or for making up keratinous substances, in the form cast as a stick, comprising:
- at least one emulsifying agent,
- at least one wax, and
- at least one ester comprising an aromatic group which is liquid at room temperature chosen from glyceryl monobenzoyl ricinoleate, glyceryl mono-dibenzoyl ricinoleate, glyceryl dibenzoyl ricinoleate, glyceryl tribenzoyl ricinoleate and their mixtures,
the said wax representing 2 to 30% of the total weight of the composition and the hardness of the said composition being between 100 and 250 g.

3. Composition according to either of the preceding claims, **characterized in that** the aromatic ester is present in an amount ranging from 0.1 to 99.9%, preferably from 1 to 99% and better still from 5 to 90%, of the total weight of the composition.

4. Composition according to one of the preceding claims, **characterized in that** it moreover comprises at least one additional fatty substance chosen from fatty substances which are liquid at room temperature, other than the said aromatic ester, pasty fatty substances, gums, resins, lipophilic polymers and their mixtures.

5. Composition according to one of the preceding claims, **characterized in that** it comprises at least one colouring material.

6. Composition according to the preceding claim, **characterized in that** the colouring material is chosen from dyes which are soluble or dispersible in the composition, pigments, pearlescent agents and their mixtures.

7. Composition according to one of the preceding claims, **characterized in that** it additionally comprises an additive chosen from antioxidants, cosmetic or dermatological active principles, preservatives, agents for gelling liquid fatty substances, dispersing agents and their mixtures.

8. Composition according to one of the preceding claims, **characterized in that** it contains a cosmetic active principle chosen from vitamins A, E, C or B₃, provitamins, such as D-panthenol, soothing active principles, such as α-bisabolol, aloe vera, allantoin, plant extracts or essential oils, protecting or restructuring agents, such as ceramides, freshness active principles, such as menthol and its derivatives, emollients (cocoa butter, dimethicone), moisturizing agents (arginine PCA), antiwrinkle active principles, essential fatty acids, and their mixtures.

9. Composition according to one of the preceding claims, **characterized in that** it is provided in the form of a lipstick.

10. Cosmetic process for caring for or making up the skin or lips of human beings, comprising the application to the skin or lips of the cosmetic composition according to one of the preceding claims.

## Patentansprüche

1. Kosmetische Zusammensetzung für die Pflege oder zum Schminken von Keratinsubstanzen in Form eines gegossenen Stiftes, die enthält:
- mindestens einen Füllstoff in Partikelform,
- mindestens ein Wachs und
- mindestens einen Ester mit aromatischer Gruppe, der bei Raumtemperatur flüssig ist und unter Glycerylinonobenzoylricinoleat, Glycerylmono-dibenzoylricinoleat, Glyceryldibenzoylricinoleat, Glyceryltribenzoylricinoleat und deren Gemischen ausgewählt ist,
wobei das Wachs 2 bis 30 % des Gesamtgewichts der Zusammensetzung ausmacht und die Härte der Zusammensetzung im Bereich von 100 bis 250 g liegt.

2. Kosmetische Zusammensetzung für die Pflege oder zum Schminken von Keratinsubstanzen in Form eines gegossenen Stiftes, die enthält:
- mindestens einen Emulgator,
- mindestens ein Wachs und
- mindestens einen Ester mit aromatischer Gruppe, der bei Raumtemperatur flüssig ist und unter Glycerylmonobenzoylricinoleat, Glycerylmono-dibenzoylricinoleat, Glyceryldibenzoylricinoleat, Glyceryltribenzoylricinoleat und deren Gemischen ausgewählt ist,
wobei das Wachs 2 bis 30 % des Gesamtgewichts der Zusammensetzung ausmacht und die Härte der Zusammensetzung im Bereich von 100 bis 250 g liegt.

3. Zusammensetzung nach einem der vorhergehenden Ansprüche, **dadurch gekennzeichnet, dass** der aromatische Ester in einer Menge von 0,1 bis 99,9 %, vorzugsweise 1 bis 99 % und besser 5 bis 90 % des Gesamtgewichts der Zusammensetzung enthalten ist.

4. Zusammensetzung nach einem der vorhergehenden Ansprüche, **dadurch gekennzeichnet, dass** sie ferner mindestens eine zusätzliche Fettsubstanz enthält, die unter den bei Raumtemperatur flüssigen Fettsubstanzen, die von dem aromatischen Ester verschieden sind, pastösen Fettsubstanzen, Gummis, Harzen, lipophilen Polymeren und deren Gemischen ausgewählt ist.

5. Zusammensetzung nach einem der vorhergehenden Ansprüche, **dadurch gekennzeichnet, dass** sie mindestens ein Farbmittel enthält.

6. Zusammensetzung nach dem vorhergehenden Anspruch, **dadurch gekennzeichnet, dass** das Farbmittel unter den in der Zusammensetzung löslichen oder dispergierbaren Farbstoffen, Pigmenten, Perlglanzpigmenten und deren Gemischen ausgewählt ist.

7. Zusammensetzung nach einem der vorhergehenden Ansprüche, **dadurch gekennzeichnet, dass** sie ferner mindestens einen Zusatzstoff enthält, der unter den Antioxidantien, kosmetischen oder dermatologischen Wirkstoffen, Konservierungsmitteln, Gelbildnern für die flüssigen Fettsubstanzen, Dispergiermitteln und deren Gemischen ausgewählt ist.

8. Zusammensetzung nach einem der vorhergehenden Ansprüche, **dadurch gekennzeichnet, dass** sie einen kosmetischen Wirkstoff enthält, der unter den Vitaminen A, E, C, B₃, Provitaminen, wie D-Panthenol, beruhigenden Wirkstoffen, wie α-Bisabolol, Aloe vera, Allantoin, Pflanzenextrakten oder etherischen Ölen, schützenden oder restrukturierenden Wirkstoffen, wie Ceramiden, erfrischenden Stoffen, wie Menthol und seinen Derivaten, Emollientien (Kakaobutter, Dimethicon), Hydratisierungsmitteln (Arginin PCA), Wirkstoffen gegen Falten, essentiellen Fettsäuren und deren Gemischen ausgewählt ist.

9. Zusammensetzung nach einem der vorhergehenden Ansprüche, **dadurch gekennzeichnet, dass** sie als Lippenstift vorliegt.

10. Kosmetisches Verfahren für die Pflege oder zum Schminken der menschlichen Haut oder der menschlichen Lippen, das das Auftragen der kosmetischen Zusammensetzung nach einem der vorhergehenden Ansprüche auf die Haut oder die Lippen umfasst.
